# EUROPÄISCHE PATENTSCHRIFT

(11) **EP 3 097 873 B1**
(45) Veröffentlichungstag und Bekanntmachung des Hinweises auf die Patenterteilung: **13.11.2019**
(21) Anmeldenummer: 16165351.4
(22) Anmeldetag: 14.04.2016
(51) Int. Cl.: A61B 17/29

(54) **WERKZEUG FÜR EIN ZERLEGBARES MEDIZINISCHES INSTRUMENT**
TOOL FOR A DISMOUNTABLE MEDICAL INSTRUMENT
OUTIL POUR UN INSTRUMENT MEDICAL DEMONTABLE

(30) Priorität: 26.05.2015 DE 102015108219
(43) Veröffentlichungstag der Anmeldung: 30.11.2016
(73) Patentinhaber: Karl Storz SE & Co. KG, 78532 Tuttlingen (DE)
(72) Erfinder: Kärcher, Daniel, 78532 Tuttlingen (DE); Stefan, Jochen, 78532 Tuttlingen (DE)

(56) Entgegenhaltungen:
- EP-A1- 2 653 110
- EP-A1- 2 653 123
- WO-A1-2010/114634

## Beschreibung

Die vorliegende Erfindung ist auf ein Werkzeug für ein zerlegbares medizinisches Instrument und auf ein zerlegbares medizinisches Instrument bezogen.

Laparoskopische und andere mikroinvasive medizinische Maßnahmen sollen durch möglichst kleine natürliche oder künstliche Körperöffnungen hindurch und in möglichst kleinen Hohlräumen möglich sein. Dafür sind möglichst dünne medizinische Instrumente erforderlich. Andererseits ist für die Reinigung und Sterilisation des Werkzeugs nach einer Verwendung und vor einer nachfolgenden Verwendung eine möglichst weitgehende Zerlegbarkeit erforderlich oder zumindest erwünscht.

Bekannte und erprobte Konzepte zur lösbaren mechanischen Verbindung mehrerer Komponenten eines zerlegbaren medizinischen Instruments sind bei fortschreitender Miniaturisierung nur begrenzt skalierbar. Deshalb müssen neue Konzepte zur Zerlegbarkeit medizinischer Instrumente entwickelt werden.

In der Patentanmeldung WO 201 0/114634 A1 ist ein zerlegbares medizinisches Instrument mit einem Werkzeug beschrieben, bei dem das Werkzeug aus zwei Bauteilen besteht. Das erste Bauteil weist dabei einen Hohlraum auf, der ein distales Endes eines Schafts aufnehmen kann. Weiterhin ist ein zweites Bauteil vorhanden, das eine Knagge umfasst, die in den beschriebenen Hohlraum ragt und eine Renkverbindung zwischen dem Schaft und dem Werkzeug, wobei die Knagge in einen Schlitz oder eine Nut am distalen Ende des Schafts eingreift.

Die Druckschrift D2, EP 2 653 110 A1 zeigt ein zerlegbares medizinisches Instrument mit unter anderem einem Werkzeug mit einer Knagge sowie einer Gelenkeinrichtung. Werkzeug und Gelenkeinrichtung sind koppelbar, wobei dazu ein weiteres Bauteil des Werkzeugs, eine Hülse ringförmig über die Knaggen wirkt.

Eine Aufgabe der vorliegenden Erfindung besteht darin, ein verbessertes, insbesondere weiter miniaturisierbares Werkzeug für ein zerlegbares medizinisches Instrument und ein verbessertes, insbesondere weiter miniaturisierbares zerlegbares medizinisches Instrument zu schaffen.

Diese Aufgabe wird durch den Gegenstand des unabhängigen Anspruchs gelöst. Weiterbildungen sind in den abhängigen Ansprüchen angegeben.

Ausführungsformen der vorliegenden Erfindung beruhen auf der Idee, an einem Werkzeug für ein zerlegbares medizinisches Instrument Knaggen für eine Renkverbindung mit dem distalen Ende eines Schafts nicht nach außen, sondern nach innen ragend vorzusehen. Die Knaggen sind folglich dafür vorgesehen, nicht von innen, sondern von außen in Schlitze oder Nuten am distalen Ende eines Schafts einzugreifen. Das dabei auftretende fertigungstechnische Problem wird insbesondere gelöst, indem die Knaggen nicht zusammen mit einem Bauteil, das einen Hohlraum zur Aufnahme des distalen Endes eines Schafts bildet, gefertigt werden. Vielmehr sind in dem Bauteil zunächst nur Ausnehmungen für die Knaggen vorgesehen, in die die Knaggen dann eingesetzt und in denen sie befestigt werden.

Werkzeug für ein zerlegbares medizinisches Instrument umfasst ein erstes Bauteil, das einen Hohlraum zur Aufnahme eines distalen Endes eines Schafts umgibt, und ein zweites Bauteil mit mehreren Knaggen, die über den inneren Umfang des Hohlraums gleichmäßig bzw. mit gleichen Winkelabständen verteilt sind, die von der kreiszylindermantelförmigen Oberfläche des Hohlraums aus in radialer Richtung in den Hohlraum ragen und zur Bildung einer Renkverbindung vorgesehen und ausgebildet sind, um von außen in Schlitze oder nach außen offene Nuten am in den Hohlraum ragenden distalen Ende des Schafts einzugreifen, wobei das erste Bauteil Ausnehmungen aufweist, in die das zweite Bauteil gefügt ist, wobei ein radial äußeres Ende oder ein radial äußerer Bereich jeder Knagge eine jeweilige Ausnehmung der Ausnehmungen vollständig ausfüllt.

Das Werkzeug ist insbesondere für ein zerlegbares medizinisches Instrument für mikroinvasive humanmedizinische oder veterinärmedizinische Maßnahmen vorgesehen und ausgebildet. Das Werkzeug kann vorgesehen und ausgebildet sein, um am distalen Ende eines medizinischen Instruments durch einen Arbeitskanal in einem Endoskop oder einem anderen medizinischen Instrument hindurchgeführt zu werden.

Das erste Bauteil ist insbesondere monolithisch, d. h. in einem Stück durch Gießen, Sintern, Schmieden oder aus einem Stück durch Fräsen, Wasserstrahlschneiden, Laserschneiden, Erodieren oder auf andere Weise in einem Stück hergestellt. Der Hohlraum zur Aufnahme eines distalen Endes eines Schafts ist insbesondere am proximalen Ende oder nahe dem proximalen Ende des Werkzeugs angeordnet. Der Hohlraum ist insbesondere vom proximalen Ende des Werkzeugs her zugänglich, so dass das distale Ende eines Schafts von proximal her in den Hohlraum eingeführt werden kann. Der Hohlraum weist insbesondere die Gestalt einer kreiszylindrischen oder im wesentlichen kreiszylindrischen Sackbohrung und eine Mantelfläche in der Gestalt der Mantelfläche eines Kreiszylinders auf.

Das zweite Bauteil weist mehrere Knaggen auf, die über den inneren Umfang des Hohlraums gleichmäßig bzw. mit gleichen Winkelabständen verteilt sind. Die Knaggen ragen von der kreiszylindermantelförmigen Oberfläche des Hohlraums aus in radialer Richtung in den Hohlraum hinein. Die Knaggen sind vorgesehen und ausgebildet, um von außen in Schlitze oder nach außen offene Nuten am distalen Ende eines Schafts einzugreifen.

Renkverbindungen werden oft auch als Bajonettverbindungen bezeichnet. Eine Renkverbindung wird insbesondere durch eine translatorische Bewegung des Werkzeugs in einer vorbestimmten Richtung bzw. parallel zu einer vorbestimmten Achse relativ zum distalen Ende eines Schafts und eine nachfolgende Rotation des Werkzeugs um die vorbestimmte Achse relativ zum distalen Ende des Schafts hergestellt. In diesem Fall weist jeder der Schlitz oder jede Nut am distalen Ende des Schafts insbesondere eine L-förmige Gestalt auf. Bei der Translationsbewegung wird jede Knagge durch einen ersten, sich parallel oder im Wesentlichen parallel zur Längsachse des Schafts erstreckende Schenkel des Schlitzes oder der Nut bis zu einem Übergang des ersten Schenkels in einen dazu im Wesentlichen orthogonalen zweiten Schenkel bewegt. Der zweite Schenkel des Schlitzes oder der Nut erstreckt sich insbesondere in Richtung des Umfangs des Schafts. Bei der Rotation des Werkzeugs relativ zum distalen Ende des Schafts wird die Knagge in den zweiten Schenkel des Schlitzes oder der Nut hineinbewegt. Bei dem so erreichten Zustand verbindet der Formschluss zwischen der Knagge und dem zweiten Schenkel des Schlitzes oder der Nut das Werkzeug mit dem Schaft derart, dass diese nicht durch eine einfache Translationsbewegung voneinander getrennt werden können.

Das zweite Bauteil kann die Knaggen umfassen oder lediglich aus Knaggen bestehen. Ein Teil des zweiten Bauteil, ein radial äußeres Ende oder ein radial äußerer Bereich jeder Knagge, füllt eine jeweilige Ausnehmung der Ausnehmungen vollständig aus. Das zweite Bauteil ist mit den Ausnehmungen bzw. dem Rand der Ausnehmungen insbesondere form- und stoffschlüssig gefügt.

Die Ausgestaltung des Werkzeugs derart, dass ein Hohlraum zur Aufnahme eines distalen Endes eines Schafts an einem ersten Bauteil und die Knaggen zur Bildung einer Renkverbindung an einem zweiten Bauteil vorgesehen und diese beiden Bauteile dann miteinander starr verbunden werden, kann eine weitgehende Miniaturisierung des Werkzeugs ermöglichen. Insbesondere bei Durchmessern des Werkzeugs und des Schafts im Bereich von 2 mm oder weniger ist eine Anformung einer Knagge unmittelbar am ersten Bauteil durch Fräsen, Erodieren oder auf andere Weise allenfalls mit sehr hohem Aufwand möglich. Die Ausgestaltung der Knaggen an einem zweiten Bauteil schafft Spielraum für eine weitere Miniaturisierung bis zu einem Durchmesser des Werkzeugs von 1,6 mm oder weniger.

Bei einem Werkzeug, wie es hier beschrieben ist, umfasst das erste Bauteil insbesondere ein Lager für die bewegbare Lagerung einer Branche oder eines anderen bewegbaren Bauteils des Werkzeugs.

Das erste Bauteil ist insbesondere ein Gabelbauteil, das zwei parallele oder im Wesentlichen parallele Holme umfasst. Zwischen den distalen Enden der Holme ist insbesondere eine Welle vorgesehen, die mit dem Gabelbauteil monolithisch gefertigt sein kann. Alternativ können die Enden der Welle in Bohrungen oder andere Ausnehmungen in den distalen Enden der Holme des Gabelbauteils befestigt oder gelagert sein. Die bewegbare Branche oder der andere bewegbare Bauteil des Werkzeugs ist insbesondere um eine durch die Welle definierte Schwenkachse schwenkbar. Das Werkzeug umfasst insbesondere zwei bewegbare Branchen, die gegenläufig um die durch die Welle definierte Schwenkachse schwenkbar sind.

Durch die Bildung eines Lagers am ersten Bauteil kann das Werkzeug - von einer oder mehreren bewegbaren Branchen und Einrichtungen zu deren Bewegung abgesehen - lediglich aus dem ersten Bauteil und dem zweiten Bauteil gebildet sein. Dieser einfache Aufbau begünstigt die Miniaturisierung und senkt die Fertigungskosten.

Bei einem Werkzeug, wie es hier beschrieben ist, ist jede Ausnehmung am ersten Bauteil insbesondere ein nach proximal offener Schlitz, in den eine Knagge von proximal eingeführt ist, und in dem die Knagge befestigt ist.

Die Knagge umfasst insbesondere einen radial äußeren Bereich, der den Schlitz ausfüllt, und einen radial inneren Bereich, der in den vom ersten Bauteil umgebenen Hohlraum ragt.

Bei einem Werkzeug, wie es hier beschrieben ist, sind die Knaggen insbesondere mit dem Rand der Ausnehmungen stoffschlüssig verbunden.

Die Knaggen sind mit dem Rand der Ausnehmungen insbesondere mittels Laserschweißens oder mittels eines anderen Schweißverfahrens verbunden.

Bei einem Werkzeug, wie es hier beschrieben ist, umfasst das zweite Bauteil insbesondere die Knaggen und einen ringförmigen Bereich, wobei der ringförmige Bereich proximal der Knaggen angeordnet ist.

Bei einem Werkzeug, wie es hier beschrieben ist, ist insbesondere der ringförmige Bereich des zweiten Bauteils mit einem proximalen Rand des ersten Bauteils gefügt.

Die Ausgestaltung des zweiten Bauteils mit einem ringförmigen Bereich, an dem mehrere Knaggen vorgesehen sind, kann die Fertigung und die Handhabung des zweiten Bauteils vereinfachen. Insbesondere kann der ringförmige Bereich auf einfache Weise mit dem proximalen Rand des ersten Bauteils ausgerichtet und optional anschließend gefügt werden.

Ein Werkzeug, wie es hier beschrieben ist, umfasst insbesondere ferner eine Übertragungseinrichtung zum Übertragen einer Kraft zu einer Branche oder einem anderen bewegbaren Bauteil des Werkzeugs und einen Verriegelungsvorsprung an der Übertragungseinrichtung zum Eingreifen in Schlitze oder Nuten an einem in den Hohlraum im ersten Bauteil eingeführten distalen Ende eines Schafts, zum Verriegeln einer Renkverbindung zwischen dem Werkzeug und dem Schaft.

Die Übertragungseinrichtung umfasst insbesondere eine Stange oder ein Rohr zur Anordnung im Inneren eines Schafts, deren proximales Ende mit einer Betätigungseinrichtung zur manuellen Bewegung und Ausübung einer Kraft koppelbar ist. Der Verriegelungsvorsprung ist insbesondere ein in Längsrichtung bzw. parallel zur vorgesehenen Bewegungsrichtung der Übertragungseinrichtung angeordneter Steg nahe dem distalen Ende der Übertragungseinrichtung. Der Verriegelungsvorsprung ist insbesondere zum Eingreifen in einen in Längsrichtung bzw. parallel zur vorgesehenen Bewegungsrichtung der Übertragungseinrichtung sich erstreckenden Abschnitt des gleichen Schlitzes vorgesehen, in den auch die Knaggen eingreifen.

Bei einem Werkzeug, wie es hier beschrieben ist, sind der Verriegelungsvorsprung und die Knaggen insbesondere dazu ausgebildet, von radial entgegengesetzten Seiten aus in Schlitze eines in den Hohlraum im ersten Bauteil eingeführten distalen Endes eines Schafts einzugreifen. Dabei sind die Knaggen und der Verriegelungsvorsprung dazu ausgebildet, von distal in die Schlitze einzugreifen.

Insbesondere ist der Verriegelungsvorsprung dazu vorgesehen und ausgebildet, um von innen in die Schlitze eines in den Hohlraum im ersten Bauteil eingeführten distalen Endes eines Schafts einzugreifen, wobei die Knaggen von außen in die Schlitze eingreifen.

Bei einem Werkzeug, wie es hier beschrieben ist, umfasst die Übertragungseinrichtung insbesondere einen Stab oder ein Rohr mit einem Schlitz am distalen Ende des Stabs oder Rohrs und ein Endstück mit einem Lager am distalen Ende des Endstücks für eine gelenkige mechanische Kopplung mit einem bewegbaren Bauteil des Werkzeugs, wobei ein proximales Ende des Endstücks in den Schlitz am distalen Ende des Stabs oder Rohrs eingesetzt und mit diesem gefügt ist, und wobei ein Bereich des proximalen Endes des Endstücks, der seitlich aus dem Schlitz am distalen Endes des Stabs oder Rohrs herausragt, den Verriegelungsvorsprung bildet.

Das Lager am distalen Ende des Endstücks ist insbesondere vorgesehen zur gelenkigen Verbindung mit einem oder mehreren Pleueln, deren zweite Enden jeweils gelenkig mit einer Branche oder einem anderen bewegbaren Bauteil des Werkzeugs gelenkig verbunden sind. Durch ein Pleuel wird eine Translationsbewegung der Übertragungseinrichtung mit einer Schwenkbewegung einer Branche oder eines anderen bewegbaren Bauteils des Werkzeugs gekoppelt. Alternativ könnte die Branche oder könnte das andere bewegbare Bauteil des Werkzeugs auch ohne die Pleueln mit dem Lager am distalen Ende des Endstücks verbunden sein.

Das proximale Ende des Endstücks ist insbesondere dünn und plattenförmig ausgebildet. Die seitlichen Ränder des dünnen plattenförmigen proximalen Endes des Endstücks weisen insbesondere rechteckige oder halbkreisförmige Querschnitte auf. Die Breite des dünnen plattenförmigen proximalen Endes des Endstücks ist insbesondere größer als der Durchmesser des Stabs oder Rohrs der Übertragungseinrichtung, so dass ein oder beide seitlichen Ränder des dünnen plattenförmigen proximalen Endes des Endstücks aus dem Schlitz im distalen Ende des Stabs oder Rohrs herausragen, um einen bzw. zwei Verriegelungsvorsprünge zu bilden.

Alternativ weist der Stab oder das Rohr der Übertragungseinrichtung am distalen Ende eine Nut auf, so dass die Mantelfläche des Stabs oder Rohrs nicht an zwei gegenüberliegenden Seiten, sondern lediglich an einer Stelle streifenförmig unterbrochen ist. In diesem Fall ragt das dünne plattenförmige proximale Ende des Endstücks nur an einer Seite aus dem Stab oder Rohr der Übertragungseinrichtung heraus, um einen Verriegelungsvorsprung zu bilden.

Der beschriebene Aufbau der Übertragungseinrichtung aus nur zwei Bestandteilen, nämlich dem Stab oder Rohr und dem Endstück, die jedoch gleichzeitig den Verriegelungsvorsprung bilden, ermöglicht einen vergleichsweise einfachen Aufbau und eine kostengünstige Fertigung.

Ein Schaft für ein medizinisches Werkzeug umfasst ein Schaftrohr, L-förmige Schlitze oder L-förmige Nuten am distalen Ende des Schaftrohrs und ein Stützrohr am distalen Ende des Schafts zum mechanischen Stützen und Verstärken des distalen Endes des Schaftrohrs, wobei das Stückrohr innerhalb des Schaftrohrs angeordnet ist.

An herkömmlichen Schäften für medizinische Werkzeuge sind Stützrohre in der Regel außen am Schaftrohr angeordnet, wobei die Knaggen eines Werkzeugs von innen in Nuten oder Schlitze im Schaftrohr eingreifen. Die Anordnung des Stützrohrs innerhalb des Schaftrohrs ermöglicht Eingreifen der Knaggen von außen in die L-förmigen Schlitze oder in die L-förmige Nuten am distalen Ende des Schaftrohrs.

Ein zerlegbares medizinisches Instrument umfasst einen Schaft und ein Werkzeug, wie es hier beschrieben ist.

Bei einem medizinischen Instrument, wie es hier beschrieben ist, umfasst der Schaft insbesondere ein Schaftrohr, L-förmige Schlitze oder L-förmige Nuten am distalen Ende des Schaftrohrs und ein Stützrohr am distalen Ende des Schafts zum mechanischen Stützen und Verstärken des distalen Endes des Schaftrohrs, wobei das Stützrohr innerhalb des Schaftrohrs angeordnet ist.

Bei einem Schaft, wie er hier beschrieben ist, oder einem medizinischen Instrument, wie es hier beschrieben ist, weist das Stützrohr insbesondere Schlitze oder Nuten zur Aufnahme des Verriegelungsvorsprungs an der Übertragungseinrichtung auf.

Die Schlitze oder die Nuten im Stützrohr können gerade oder L-förmig ausgebildet sein Die Schlitze oder die Nuten im Stützrohr sind insbesondere vollständig oder abschnittsweise parallel zur Längsachse des Schafts und zur vorgesehenen Bewegungsrichtung der Übertragungseinrichtung des Werkzeugs.

Die Schlitze oder die Nuten im Stützrohr können teilweise oder vollständig deckend mit den L-förmigen Schlitze oder den L-förmigen Nuten am distalen Ende des Schaftrohrs angeordnet sein. Insbesondere sind die Schlitze oder die Nuten im Stützrohr gerade und parallel und teilweise oder vollständig deckend mit dem oben erwähnten ersten Abschnitt bzw. ersten Schenkel der L-förmigen Schlitze oder der L-förmigen Nuten am distalen Ende des Schaftrohrs angeordnet. Eine deckungsgleiche Anordnung einander entsprechender Schlitze im Stützrohr und im Schaftroh kann ein besonders tiefes Eingreifen des Verriegelungsvorsprungs und/oder der Knaggen du und damit einen besonders guten Formschluss ermöglichen.

Alternativ können die insbesondere geraden - Schlitze oder die - insbesondere geraden - Nuten im Stützrohr gegenüber den L-förmigen Schlitze oder den L-förmigen Nuten versetzt angeordnet sein, um eine besonders hohe Steifheit des distalen Endes des Schafts zu erzielen.

Im Falle zweier Schlitze oder Nuten am distalen Ende des Schaftrohrs und/oder zweier Schlitze oder Nuten im Stützrohr sind die Schlitze oder Nuten im Schaftrohr und die Schlitze oder Nuten im Stützrohr beispielsweise abwechselnd mit Winkelabständen von ca. 90 Grad angeordnet.

### Kurzbeschreibung der Figuren

Nachfolgend werden Ausführungsformen anhand der beigefügten Figuren näher erläutert.

Es zeigen:
- Figur 1: eine schematische axonometrische Darstellung eines distalen Endes eines medizinischen Instruments;
- Figur 2: eine schematische axonometrische Darstellung eines Werkzeugs des medizinischen Instruments aus Figur 1;
- Figur 3: eine schematische axonometrische Darstellung von Bestandteilen eines Schafts des medizinischen Instruments aus Figur 1;
- Figur 4: eine weitere schematische axonometrische Darstellung von Teilen des Werkzeugs aus den Figuren 1 und 2;
- Figur 5: eine weitere schematische axonometrische Darstellung von Teilen des Werkzeugs aus den Figuren 1 und 2;
- Figur 6: eine schematische Darstellung eines Teils des Werkzeugs aus den Figuren 1, 2, 4 und 5;
- Figur 7: eine weitere schematische axonometrische Darstellung von Teilen des Werkzeugs aus den Figur 1 und 2;
- Figur 8: eine weitere schematische Darstellung eines Teils des Werkzeugs aus den Figuren 1 und 2;
- Figur 9: eine weitere schematische Darstellung des Teils aus Figur 9.

### Beschreibung der Ausführungsformen

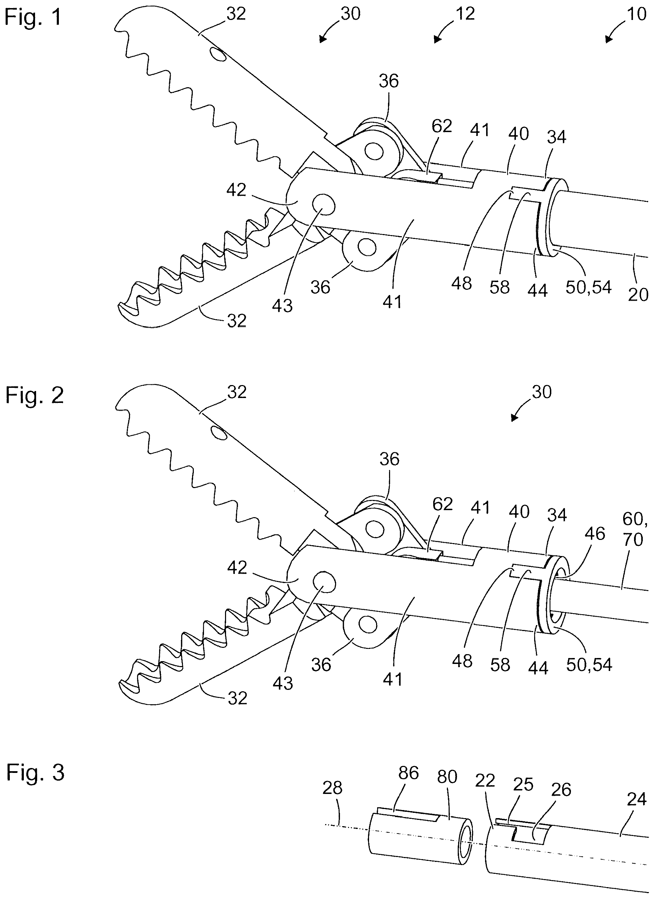

Figur 1 zeigt eine schematische axonometrische Darstellung eines distalen Endes 12 eines medizinischen Instruments 10 für mikroinvasive human- oder veterinär-medizinische Anwendungen. Das medizinische Instrument 10 weist einen langen, dünnen, starren oder flexiblen (d. h. elastisch oder zerstörungsfrei plastisch verformbaren) geraden oder gekrümmten Schaft 20 auf. Das distale des Schafts 20 ist mit einem Werkzeug 30, insbesondere mit dessen proximalen Ende 34 zerstörungsfrei lösbar mechanisch verbunden. Am in Figur 1 nicht dargestellten proximalen Ende des Schafts 20 kann eine Handhabungseinrichtung zur manuellen Handhabung des medizinischen Instruments 10 vorgesehen sein.

Das Werkzeug 30 umfasst zwei um dieselbe Schwenkachse gegenläufig schwenkbare Branchen 32 zum Greifen, Quetschen, elektrochirurgischen Koagulieren oder Schneiden von Gewebe. Das Werkzeug 30 umfasst ein erstes Bauteil bzw. Gabelbauteil 40 und ein zweites Bauteil bzw. Knaggenbauteil 50. Das Gabelbauteil 40 umfasst zwei parallele Holme 41, deren distale Enden 42 das distale Ende des Gabelbauteils 40 bilden. An den distalen Enden 42 der Holme 41 ist ein Lager 43 für die schwenkbaren Branchen 32 vorgesehen. Das Lager 43 wird durch eine die distalen Enden 42 der Holme 41 verbindende Welle gebildet. Die Welle definiert die gemeinsame Schwenkachse der schwenkbaren Branchen 32.

Das proximale Ende des Gabelbauteils 40 wird durch einen im Wesentlichen kreisförmigen proximalen Rand 44 des Gabelbauteils 40 gebildet. Der proximale Rand 44 des Gabelbauteils 40 ist durch zwei einander gegenüber angeordnete Schlitze 48 unterbrochen, von denen in Figur 1 nur einer sichtbar ist. Die Schlitze 48 weisen jeweils eine im Wesentlichen rechteckige Gestalt auf.

Das Knaggenbauteil 50 umfasst einen ringförmigen Bereich 54, der parallel zum proximalen Rand 44 des Gabelbauteils 40 angeordnet ist und mit diesem gefügt, insbesondere durch Schweißen verbunden sein kann. Das Knaggenbauteil 50 umfasst ferner zwei einander gegenüberliegende Knaggen, deren radial äußere Enden bzw. Bereiche 58 die Schlitze 48 am proximalen Ende des Gabelbauteils 40 ausfüllen und mit diesen gefügt sind. Insbesondere sind die Ränder der radial äußeren Bereiche 58 der Knaggen des Knaggenbauteils 50 mit den Rändern der Schlitze 48 durch Schweißen oder Löten verbunden.

Im Schaft 20 und im Gabelbauteil 40 des Werkzeugs 30 ist eine Übertragungseinrichtung angeordnet, die bei der Darstellung in Figur 1 weitgehend durch den Schaft 20 und das Gabelbauteil 40 des Werkzeugs 30 verdeckt ist. Lediglich ein das distale Ende der Übertragungseinrichtung bildendes Endstück 62 ist in Figur 1 zwischen den Holmen 41 des Gabelbauteils 40 des Werkzeugs 30 teilweise sichtbar. Die Übertragungseinrichtung ist innerhalb des Schafts 20 und des Gabelbauteils 40 des Werkzeugs 30 in Richtung parallel zur Längsachse des Schafts 20 innerhalb eines vorbestimmten Bereichs verschiebbar. Das proximale Ende der Übertragungseinrichtung ist beispielsweise mit einem schwenkbaren Teil der erwähnten Handhabungseinrichtung am proximalen Ende des Schafts 20 gekoppelt. Das das distale Ende der Übertragungseinrichtung bildende Endstück 62 ist über je ein Pleuel 36 mit den Branchen 32 derart gelenkig gekoppelt, dass eine Translationsbewegung der Übertragungseinrichtung mit einer Schwenkbewegung der Branchen 32 des Werkzeugs 30 einhergeht.

Figur 2 zeigt eine schematische axonometrische Darstellung des Werkzeugs 30 aus Figur 1. Die Art der Darstellung, insbesondere die Blickrichtung, entspricht weitgehend derjenigen der Figur 1.

In Figur 2 ist nur das Werkzeug 30 dargestellt, nicht der Schaft 20 des medizinischen Instruments. Deshalb ist in Figur 2 ein weiterer Teil der Übertragungseinrichtung 60 sichtbar, nämlich ein Stab 70, der zur Anordnung im Schaft 20 des medizinischen Instruments 10 (vgl. Figur 1) vorgesehen ist.

Ferner sind in Figur 2 die ringförmige Struktur des ringförmigen Bereichs 54 und ein Hohlraum 46 im Gabelbauteil 40 erkennbar.

Figur 3 zeigt eine schematische axonometrische Darstellung von Bestandteilen des Schafts 20 des medizinischen Instruments 10 aus Figur 1. Die Art der Darstellung, insbesondere die Blickrichtung, entspricht weitgehend derjenigen der Figuren 1 und 2.

Der Schaft umfasst ein Schaftrohr 24, dessen Position in Figur 3 in horizontaler Richtung der Position in Figur 1 entspricht. Am distalen Ende 22 des Schaftrohrs 24 sind zwei einander gegenüber angeordnete, jeweils L-förmige Schlitze vorgesehen, von denen in Figur 3 nur einer sichtbar ist. Jeder L-förmige Schlitz umfasst einen ersten Abschnitt 25, der sich parallel zur Längsachse 28 des Schaftrohrs 24 erstreckt, und einen zweiten Abschnitt 26, der sich in Richtung parallel zum Umfang des Schaftrohrs 24 erstreckt.

In Figur 3 ist ferner ein Stützrohr 80 dargestellt, das beim fertiggestellten und in der vorgesehenen Weise verwendbaren Schaft am distalen Ende 22 des Schafts innerhalb des Schaftrohrs 24 angeordnet ist. Das Stützrohr 80 ist in Figur 3 außerhalb des Schaftrohrs 24, nämlich in Richtung parallel zur Längsachse 28 des Schafts aus dem Schaftrohr 24 nach distal herausgezogen, dargestellt. Beim fertiggestellten und in der vorgesehenen Weise verwendbaren Schaft ist das Stützrohr 80 insbesondere derart im Schaftrohr 24 angeordnet, dass der distale Rand des Stützrohrs 80 und der distale Rand des Schaftrohrs 24 in einer Ebene liegen.

Das Stützrohr 80 weist zwei gerade, jeweils rechteckige oder im Wesentlichen rechteckige und einander gegenüberliegende Schlitze 86 auf, von denen in Figur 3 nur einer sichtbar ist. Die Schlitze 86 sind parallel zur Längsachse 28 des Schafts und damit auch parallel zur vorgesehenen Bewegungsrichtung der Übertragungseinrichtung 60 (vgl. Figur 2).

Bei der vorgesehenen Anordnung des Stützrohrs 80 im Schaftrohr 24 sind die Schlitze 86 im Stützrohr 80 einerseits und die ersten Abschnitte 25 der L-förmigen Schlitze im Schaftrohr 24 andererseits miteinander in Deckung oder im Wesentlichen in Deckung. Beim fertiggestellten Schaft 20 ist das Stützrohr 80 mit dem Schaftrohr 24 insbesondere stoffschlüssig (beispielsweise durch Laserschweißen oder Löten) und optional zusätzlich reib- bzw. kraftschlüssig verbunden. Das Stützrohr 80 verstärkt das distale Ende 22 des Schafts. Ferner entspricht der innere Querschnitt des Stützrohrs 80 (von den Schlitzen 86 abgesehen) weitgehend dem äußeren Querschnitt des Stabs 70 der Übertragungseinrichtung 60 (vgl. Figur 2), so dass die Übertragungseinrichtung 60 im Stützrohr 80 spiel- und reibungsarm geführt ist.

Ferner entspricht der äußere Querschnitt des Schaftrohrs 24 und damit des Schafts an dessen distalem Ende 22 (von den L-förmigen Schlitzen 25, 26 abgesehen) dem Querschnitt des Hohlraums 46 (von den Knaggen abgesehen) am proximalen Ende des Gabelbauteils 40 (vgl. Figuren 2, 5, 6), so dass das distale Ende 22 des Schafts 20 im Hohlraum 46 im Gabelbauteil 40 spielarm geführt ist.

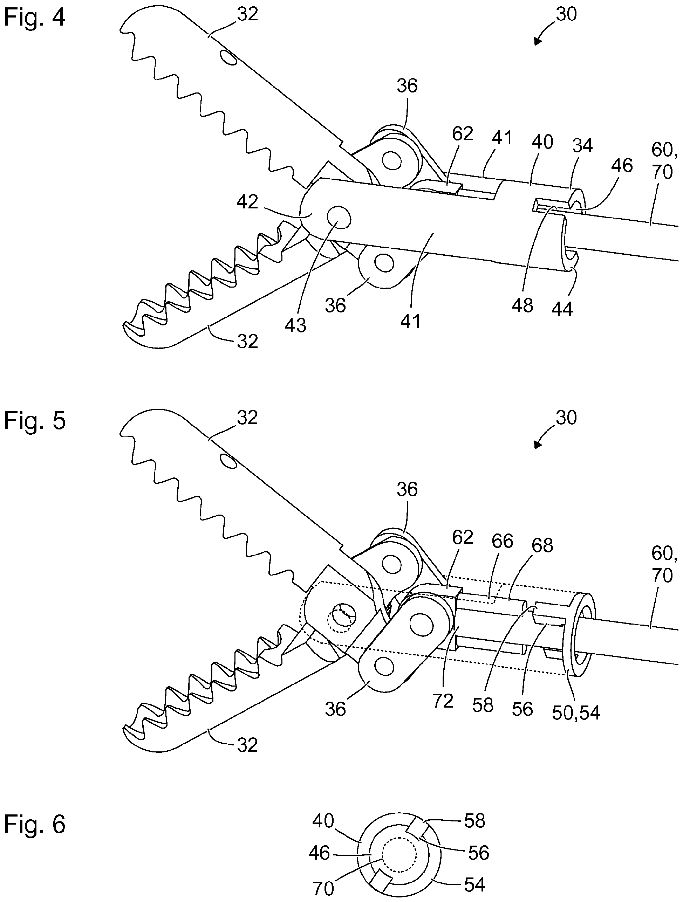

Figur 4 zeigt eine weitere schematische axonometrische Darstellung von Teilen des Werkzeugs 30 aus Figur 2. Die Art der Darstellung, insbesondere die Blickrichtung, entspricht weitgehend derjenigen der Figuren 1 und 2.

In Figur 4 ist das Werkzeug 30 ohne das Knaggenbauteil 50 dargestellt. Deshalb sind die Schlitze 48 im Gabelbauteil 40, die vom im Wesentlichen kreisförmigen proximalen Rand 44 des Gabelbauteils 40 ausgehen und diesen unterbrechen, sichtbar.

Figur 5 zeigt eine weitere schematische axonometrische Darstellung des Werkzeugs 30 aus den Figuren 2 und 4. Die Art der Darstellung, insbesondere die Blickrichtung, entspricht weitgehend derjenigen der Figuren 1, 2 und 4.

In Figur 5 ist das Gabelbauteil 40 des Werkzeugs 30 transparent dargestellt und lediglich durch seine bei der gewählten Blickrichtung sichtbaren Konturen angedeutet. Deshalb sind in Figur 5 die Knaggen 56 distal des ringförmigen Bereichs 54 des Knaggenbauteils 50 sichtbar, deren äußere Enden bzw. Bereiche 58 die Schlitze 48 im Gabelbauteil 40 (vgl. Figur 4) ausfüllen. Die radial inneren Enden der Knaggen 56 reichen in den Hohlraum 46 am proximalen Ende des Gabelbauteils 40 hinein (vgl. Figur 4).

Ferner ist in Figur 5 die Übertragungseinrichtung 60 weitgehend sichtbar. Die Übertragungseinrichtung 60 umfasst einen Stab 70 und ein Endstück 62, das das distale Ende der Übertragungseinrichtung 60 bildet. Das Endstück 62 umfasst ein distales Ende 64 mit einem Lager zur gelenkigen Verbindung mit den Pleueln 36 und ein im Wesentlichen dünnes und plattenförmiges proximales Ende 66, das in einem Schlitz am distalen Ende 72 des Stabs 70 angeordnet ist. Aus dem Schlitz am distalen Ende 72 des Stabs 70 seitlich heraus ragende Ränder des dünnen plattenförmigen proximalen Endes 66 des Endstücks 62 bilden zwei stegförmige Verriegelungsvorsprünge 68. Die Verriegelungsvorsprünge 68 erstrecken sich jeweils parallel zur Längsachse des Stabs 70 der Übertragungseinrichtung 60 und damit auch parallel zur Längsachse 28 des Schafts 20 (vgl. Figur 3) und zu den Schlitzen 86 im Stützrohr 80.

Figur 6 zeigt eine schematische Darstellung des Knaggenbauteils 50 in einer Ansicht von distal. Zur Orientierung ist in gestrichelter Linie auch die Kontur des Querschnitts des Stabs 70 der Übertragungseinrichtung 60 (vgl. Figur 5) angedeutet.

Die innere Kontur des ringförmigen Bereichs 54 des Knaggenbauteils 50 entspricht der Kontur des Querschnitts des Hohlraums 46 am proximalen Ende des Gabelbauteils 40 (vgl. Figuren 2, 4). Die Knaggen 56 greifen von außen in den Hohlraum 46 ein.

Die Breiten der Knaggen 56 entsprechen den Breiten der ersten Abschnitte 25 der L-förmigen Schlitze im Schaftrohr 24, so dass die Knaggen reibungsarm durch die ersten Abschnitte 25 der L-förmigen Schlitze im Schaftrohr 24 hindurch geführt werden können. Wenn die Knaggen 56 so tief in den Hohlraum 46 eingreifen, dass sie auch in die Schlitze 86 im Stützrohr 80 (vgl. Figur 3) eingreifen, sind auch die Schlitze 86 im Stützrohr 80 mindestens so breit wie die Knaggen 56 und abweichend von der Darstellung in Figur 3 L-förmig.

Die Längen der Knaggen (jeweils in Richtung parallel zur Längsachse 28 des Schafts gemessen; vgl. Figur 3), entsprechen den Abmessungen der zweiten Abschnitte 26 der L-förmigen Schlitze im Schaftrohr 24, so dass die Knaggen 56 reibungsarm in die zweiten Abschnitte 26 der L-förmigen Schlitze eingeführt werden können.

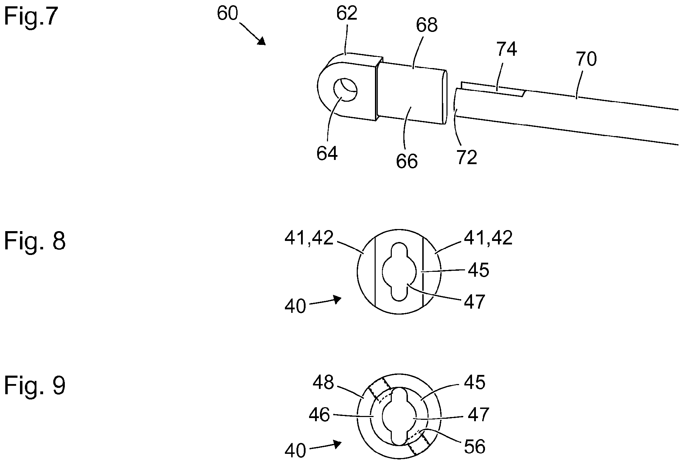

Figur 7 zeigt eine weitere schematische axonometrische Darstellung von Bestandteilen der Übertragungseinrichtung 60 des Werkzeugs 30 aus den Figuren 1 und 2. Das Endbauteil 62 ist in Figur 7 gegenüber dem Stab 70 nach distal verschoben dargestellt. Deshalb sind in Figur 7 der Schlitz 74 am distalen Ende 72 des Stabs 70 einerseits und das dünne plattenförmige proximale Ende 66 des Endstücks 62 andererseits deutlich erkennbar. Die seitlichen bzw. parallel zur Längsachse des Stabs 70 verlaufenden Kanten des proximalen Endes 66 des Endstücks 62 bilden die Verriegelungsvorsprünge 68. Bei dem dargestellten Beispiel weisen die Verriegelungsvorsprünge 68 abgerundete Querschnitte auf.

Figur 8 zeigt eine schematische Darstellung des Gabelbauteils 40 des Werkzeugs 30 aus den Figuren 1 und 2 in einer Ansicht von distal.

Das Gabelbauteil 40 weist zwischen den proximalen Enden der Holme 41 einerseits und dem distalen Ende des Hohlraums 46 (vgl. Figur 4) andererseits eine Wand 45 auf, die in den übrigen Figuren nicht sichtbar ist. Diese Wand 45 ist insbesondere im Wesentlichen plattenförmige und eben und begrenzt den Hohlraum 46 nach distal bzw. bildet die distale Oberfläche des Hohlraums 46.

In der Wand 45 ist eine Durchgangsbohrung 47 vorgesehen. Die Durchgangsbohrung 47 erstreckt sich in axialer Richtung des Gabelbauteils 40 und damit in Richtung orthogonal zur Zeichenebene der Figur 9 von dem Zwischenraum zwischen den Holmen 41 einerseits bis zum Hohlraum 46 am proximalen Ende des Gabelbauteils 40 (vgl. Figuren 2, 4), der in Figur 9 nicht sichtbar ist, andererseits.

Die Durchgangsbohrung 47 weist einen Querschnitt auf, der weitgehend dem Querschnitt der Übertragungseinrichtung 60 im Übergangsbereich zwischen dem Endstück 62 und dem Stab 70 (vgl. Figuren 5, 7) entspricht, so dass die Übertragungseinrichtung 60 in der Durchgangsbohrung 47 im Gabelbauteil 40 spiel- und reibungsarm geführt ist. Der Querschnitt der Übertragungseinrichtung 60 im Übergangsbereich zwischen dem Endstück 62 und dem Stab 70 (vgl. Figuren 5, 7) und der Querschnitt der Durchgangsbohrung 47 sind jeweils nicht kreisförmig. Insbesondere weicht der Querschnitt der Übertragungseinrichtung 60 durch die Verriegelungsvorsprünge 68 (vgl. Figuren 5) von einer reinen Kreisform ab. Dadurch ist eine Rotation der Übertragungseinrichtung 60 relativ zum Gabelbauteil 40 formschlüssig unterbunden.

Figur 9 zeigt eine weitere schematische Darstellung des Gabelbauteils 40 des Werkzeugs 30 aus den Figuren 1 und 2. In Figur 9 ist eine Ansicht des Gabelbauteils 40 von proximal gezeigt. Somit ist das Gabelbauteil 40 in den Figuren 8 und 9 aus entgegengesetzten Richtungen gezeigt.

In Figur 9 sind die Durchgangsbohrung 47, der im Wesentlichen kreiszylindrische Hohlraum 46 am proximalen Ende des Gabelbauteils 40 und die beiden einander gegenüberliegenden Schlitze 48 im Gabelbauteil 40 sichtbar. Die Konturen der in den Hohlraum 46 ragenden Knaggen 56 des in Figur 9 nicht dargestellten Knaggenbauteils 50 (vgl. Figuren 1, 2, 5 und 6) sind durch gestrichelte Linien angedeutet.

Wie bereits erwähnt ist die Übertragungseinrichtung 60 durch Formschluss zwischen dem Übergangsbereich zwischen dem Stab 70 und dem Endstück 62, insbesondere den Verriegelungsvorsprüngen 68, einerseits (vgl. Figuren 5, 7) und der Durchgangsbohrung 47 im Gabelbauteil 40 andererseits so im Gabelbauteil 40 (vgl. Figuren 8, 9) geführt ist, dass die Übertragungseinrichtung 60 gegenüber dem Gabelbauteil 40 zwar in Längsrichtung verschoben, jedoch nicht rotiert werden kann. Alternativ kann eine ähnliche Führung durch Formschluss zwischen den Pleueln 36, dem distalen Ende 64 des Endstücks 62 der Übertragungseinrichtung 60 und den einander zugewandten Flächen der Holme 41 des Gabelbauteils 40 (vgl. Figuren 2, 4) erzielt werden.

In der Zusammenschau der Figuren ist die Funktion der Bauteile und Komponenten des Werkzeugs 30 und des Schafts 20 erkennbar.

Zur lösbaren mechanischen Verbindung des Werkzeugs 30 mit dem Schaft 20 wird zunächst die Übertragungseinrichtung 60, insbesondere der Stab 70 der Übertragungseinrichtung 60 (vgl. Figur 2), von distal in den Schaft 20 (vgl. Figur 3) eingeführt.

Die Branchen 32 des Werkzeugs 30 werden in eine Überoffenstellung gebracht, bei der der Winkel zwischen den Branchen 32 größer als in den Figuren 1, 2, 4 und 5 dargestellt ist. In dieser Überoffenstellung nehmen die Verriegelungsvorsprünge 68 an der Übertragungseinrichtung 60 ihre äußerst distale Position ein, in der sie zwar in die Durchgangsbohrung 47 im Gabelbauteil 40 eingreifen, nicht jedoch in den Hohlraum 46 am proximalen Ende des Gabelbauteils 40 ragen.

Der Schaft 20 und das Werkzeug 30 werden so weit zusammengeführt, dass das distale Ende 22 des Schafts 20 vollständig in den Hohlraum 46 im Gabelbauteil 40 des Werkzeugs 30 eingeführt wird und das distale Ende 22 des Schafts 20 an der Wand 45 des Gabelbauteils 40 anliegt. Dabei werden die Knaggen 56 in die ersten Abschnitte 25 der L-förmigen Schlitze am distalen Ende 22 des Schafts 20 eingeführt. Wenn das distale Ende 22 des Schafts 20 an der Wand 45 im Gabelbauteil 40 anliegt, befinden sich die Knaggen 56 in den Übergangsbereichen zwischen den ersten Abschnitten 25 und den zweiten Abschnitten 26 der L-förmigen Schlitze am distalen Ende 22 des Schaftrohrs 24 (vgl. Figur 3).

Der Schaft 20 kann nun relativ zum Werkzeug 30 um seine Achse 28 im Uhrzeigersinn (von proximal aus betrachtet) rotiert werden. Dabei werden die Knaggen 56 von den ersten Abschnitten 25 weg in die zweiten Abschnitte 26 der L-förmigen Schlitze am distalen Ende 22 des Schafts 20 hineinbewegt.

Wenn die Knaggen 56 an den von den ersten Abschnitten 25 abgewandten Enden der zweiten Abschnitte 26 der L-förmigen Schlitze am distalen Ende 22 des Schafts 20 anliegen, befinden sich Schaft 20 und Werkzeug 30 in ihren vorgesehenen relativen Positionen, in denen Schaft 20 und Werkzeug 30 nicht durch eine einfache Translationsbewegung voneinander getrennt werden können. In dieser vorgesehenen Position des Schafts 20 relativ zum Werkzeug 30 fluchten die Verriegelungsvorsprünge 68 an der Übertragungseinrichtung 60 (vgl. Figur 5) mit den Schlitzen 86 im Stützrohr 80 und den ersten Abschnitten 25 der L-förmigen Schlitze am distalen Ende 22 des Schaftrohrs 24 (vgl. Figur 3). Die Übertragungseinrichtung 60 kann deshalb relativ zum Werkzeug 30 und zum Schaft 20 nach proximal bewegt werden, wobei die Branchen 32 ihre Überoffenstellung verlassen. Dabei greifen die Verriegelungsvorsprünge 68 an der Übertragungseinrichtung 60 in die Schlitze 86 im Stützrohr 80 und die ersten Abschnitte 25 der L-förmigen Schlitze im Schaftrohr 24 ein. Durch diesen Eingriff kann das Werkzeug 30 nicht mehr relativ zum Schaft 20 rotiert werden und die Knaggen 56 können nicht zu den ersten Abschnitten 25 der L-förmigen Schlitze im Schaftrohr 24 bewegt werden. Dadurch ist die mechanische Verbindung zwischen Schaft 20 und Werkzeug 30 verriegelt, solange die Branchen 32 sich nicht in ihrer Überoffenstellung befinden.

Durch hier nicht dargestellte Einrichtungen an den distalen Enden des Schafts 20 und der Übertragungseinrichtung 60, insbesondere in einer Handhabungseinrichtung, kann eine Bewegung in die Überoffenstellung 32 unterbunden werden.

Im spielfreien Idealfall liegen gleichzeitig einerseits der distale Rand bzw. das distale Ende 22 des Schaftrohrs 24 (vgl. Figur 3) an der Wand 45 im Gabelbauteil 40 (vgl. Figuren 8, 9) und andererseits die distalen Enden oder Ränder der Knaggen 56 (vgl. Figur 5) an den distalen Rändern der zweiten Abschnitte 26 der L-förmigen Schlitze im Schaftrohr 24 (vgl. Figur 3) an. Ein Längsspiel der mechanischen Verbindung zwischen Schaft 20 und Werkzeug 30 wird durch die Differenz zwischen dem Abstand der distalen Ränder bzw. Flanken der Knaggen 56 (vgl. Figur 5) und der Wand 45 im Gabelbauteil 40 (vgl. Figuren 8, 9) einerseits und dem Abstand zwischen den distalen Rändern der zweiten Abschnitte 26 der L-förmigen Schlitze im Schaftrohr 24 und dem distalen Ende 22 des Schaftrohrs 24 andererseits definiert.

In den Figuren 1 und 2 ist ein kleiner Abstand zwischen dem ringförmigen Bereich 54 des Knaggenbauteils 50 einerseits und dem proximalen Rand 44 des Gabelbauteils 40 andererseits angedeutet. Dieser kleine Abstand kann durch eine Schweiß- oder Lötnaht überbrückt oder aufgefüllt sein. Anders ausgedrückt sind die in Richtung parallel zur Längsachse 28 des Schafts 20 gemessenen Abmessungen der beiden Knaggen 56 etwas größer als die in der gleichen Richtung gemessenen Tiefen der Schlitze 48 im Gabelbauteil. Dadurch ist sichergestellt, dass beim Einschieben der Knaggen 56 in die Schlitze 48 die Positionen der Knaggen 56 durch den Anschlag der Knaggen 56 bzw. ihrer äußeren Bereiche 58 an den distalen Enden der Schlitze 48 im Gabelbauteil 40 formschlüssig eindeutig definiert sind. In diesen formschlüssig eindeutig definierten Positionen werden die äußeren Bereiche 58 der Knaggen 56 mit Schlitzen 48 oder mit den Rändern der Schlitze 48 gefügt, insbesondere durch Laserschweißen verbunden.

Die das mechanische Spiel zwischen Schaft 20 und Werkzeug 30 definierenden Abstände sind also jeweils an einem einzigen Bauteil definiert. Am Schaft 20 ist dies der Abstand zwischen dem distalen Rand bzw. Ende 22 des Schaftrohrs 24 und dem distalen Rand des zweiten Abschnitts 26 des L-förmigen Schlitzes im Schaftrohr 24. Am Werkzeug ist dies der Abstand zwischen der proximalen Oberfläche der Wand 45 im Gabelbauteil 40 und den distalen Enden der Schlitze 48 im Gabelbauteil, an denen die distalen Endflächen der äußeren Bereiche 58 der Knaggen 56 anliegen. Indem beide das Spiel bestimmende Abstände jeweils an einem einzigen Bauteil definiert sind, kann das Spiel besonders präzise und deshalb auch besonders klein eingestellt werden.

Zur zerstörungsfreien Lösung der mechanischen Verbindung zwischen Schaft 20 und Werkzeug 30 wird gegebenenfalls zunächst eine Blockade der Überoffenstellung an der Handhabungseinrichtung gelöst. Danach werden die Branchen 32 in ihre Überoffenstellung und die Übertragungseinrichtung 60 mit den Verriegelungsvorsprüngen 68 in ihre äußerst distale Position bewegt. In dieser Konfiguration greifen die Verriegelungsvorsprünge 68 an der Übertragungseinrichtung 60 (vgl. Figur 5) nicht mehr in die Schlitze 86 im Stützrohr 80 und in die ersten Abschnitte 25 der L-förmigen Schlitze im Schaftrohr 24 (vgl. Figur) ein. Das Werkzeug 30 kann relativ zum Schaft 20 gegen den Uhrzeigersinn (von distal aus betrachtet) rotiert werden. Dabei werden die von außen eingreifenden Knaggen 56 in den zweiten Abschnitten 26 bis zu den Übergangsbereichen zwischen den zweiten Abschnitten 26 und den ersten Abschnitten 25 der L-förmigen Schlitze im Schaftrohr 24 bewegt. Danach können Schaft 20 und Werkzeug 30 auseinandergezogen werden. Dabei werden die Knaggen 56 in den ersten Abschnitten 25 der L-förmigen Schlitze im Schaftrohr 24 bewegt und aus diesem herausgezogen.

### Bezugszeichen

- 10: medizinisches Instrument
- 12: distales Ende des medizinischen Instruments 10
- 20: Schaft des medizinischen Instruments 10
- 22: distales Ende des Schafts 20 und des Schaftrohrs 24, zur lösbaren mechanischen Verbindung mit dem proximalen Ende 34 des Werkzeugs 30
- 24: Schaftrohr des Schafts 20
- 25: erster Abschnitt eines L-förmigen Schlitzes im Schaftrohr 24, parallel zur Längsachse des Schafts 20, zum Einführen der Knagge 56 am Werkzeug 20
- 26: zweiter Abschnitt eines L-förmigen Schlitzes im Schaftrohr 24, in Richtung des Umfangs des Schafts 20, zur Aufnahme der Knagge 56 am Werkzeug 20
- 28: Längsachse des Schafts 20
- 30: Werkzeug am distalen Ende 12 des medizinischen Instruments 10
- 32: schwenkbare Branche am distalen Ende des Werkzeugs 30
- 34: proximales Ende des Werkzeugs 30 zur lösbaren mechanischen Verbindung mit dem distalen Ende 22 des Schafts 20
- 36: Pleuel zwischen Übertragungseinrichtung 60 und schwenkbarer Branche 32
- 40: erstes Bauteil bzw. Gabelbauteil des Werkzeugs 30
- 41: Holm des ersten Gabelbauteils 40
- 42: distales Ende des Gabelbauteils 40 und von dessen Holm 41
- 43: Lager am distalen Ende des Holms 41 für die schwenkbaren Branche 32
- 44: proximaler Rand des Gabelbauteils 40
- 45: Wand im Gabelbauteil 40 → auch in Beschreibung
- 46: Hohlraum im Gabelbauteil 40 am proximalen Ende des Gabelbauteils 40
- 47: Durchgangsbohrung im Gabelbauteil, dem Querschnitt der Übertragungseinrichtung 60 entsprechend
- 48: Schlitz im Gabelbauteil 40, vom proximalen Rand 44 des Gabelbauteils 40 ausgehend und insbesondere parallel zur Längsachse 28
- 50: zweites Bauteil bzw. Knaggenbauteil
- 54: ringförmiger Bereich des Knaggenbauteils 50
- 56: Knagge am Knaggenbauteil 50, in den Hohlraum 46 des Gabelbauteils ragend
- 58: äußerer Bereich der Knagge 56, in den Schlitz 48 im Gabelbauteil 40 eingesetzt
- 60: Übertragungseinrichtung zum Übertragen einer Kraft zum Werkzeug 20 zum Bewegen der schwenkbaren Branche 22
- 62: Endstück, das distale Ende der Übertragungseinrichtung 60 bildend
- 64: distales Ende des Endstücks 62, Lager
- 66: proximales Ende des Endstücks 62
- 68: Verriegelungsvorsprung an der Übertragungseinrichtung 60, gebildet durch das proximale Ende 66 des Endstücks 62
- 70: Stab der Übertragungseinrichtung 60
- 72: distales Ende des Stabs 70
- 74: Schlitz am distalen Ende 72 des Stabs 70
- 80: Stützrohr innerhalb des Schaftrohrs 20 am distalen Ende 22 des Schafts 20
- 86: Schlitz im Stützrohr 80, teilweise mit Schlitz 25, 26 am distalen Ende 22 des Schafts 20 deckend

## Patentansprüche

1. **Werkzeug** (30) für ein zerlegbares medizinisches Instrument (10), mit:
einem **ersten Bauteil** (40), das einen **Hohlraum** (46) zur Aufnahme eines distalen Endes (22) eines Schafts (20) umgibt;
einem **zweiten Bauteil** (50) mit mehreren **Knaggen** (56), die über den inneren Umfang des Hohlraums (46) gleichmäßig bzw. mit gleichen Winkelabständen verteilt sind, die von der kreiszylindermantelförmigen Oberfläche des Hohlraums (46) aus in radialer Richtung in den Hohlraum (46) ragen und zur Bildung einer Renkverbindung vorgesehen und ausgebildet sind, um von außen in Schlitze (25, 26) oder nach außen offene Nuten am in den Hohlraum (46) ragenden distalen Ende des Schafts (20) einzugreifen,
wobei das erste Bauteil (40) Ausnehmungen (48) aufweist, in die das zweite Bauteil (50) gefügt ist, wobei ein radial äußeres Ende oder ein radial äußerer Bereich (58) jeder Knagge eine jeweilige Ausnehmung (48) der Ausnehmungen (48) vollständig ausfüllt.

2. Werkzeug (30) nach dem vorangehenden Anspruch, bei dem das erste Bauteil (40) ein **Lager** (43) für die bewegbare Lagerung einer Branche (32) oder eines anderen bewegbaren Bauteils des Werkzeugs (30) umfasst.

3. Werkzeug (30) nach einem der vorangehenden Ansprüche, bei dem die Ausnehmungen (48) am ersten Bauteil (40) nach proximal offene **Schlitze** sind, in denen die Knaggen (56) von proximal eingeführt sind, und in denen die Knaggen (56) befestigt sind.

4. Werkzeug (30) nach einem der vorangehenden Ansprüche, bei dem die Knaggen (56) mit dem Rand der Ausnehmungen (48) stoffschlüssig verbunden sind.

5. Werkzeug (30) nach einem der vorangehenden Ansprüche, bei dem
das zweite Bauteil (50) die **Knaggen** (56) und einen **ringförmigen Bereich** (54) umfasst,
der ringförmige Bereich (54) proximal den Knaggen (56) angeordnet ist.

6. Werkzeug (30) nach dem vorangehenden Anspruch, bei dem der **ringförmige Bereich** (54) des zweiten Bauteils (50) mit einem **proximalen Rand** (44) des ersten Bauteils (40) gefügt ist.

7. Werkzeug (10) nach einem der vorangehenden Ansprüche, ferner mit:
einer **Übertragungseinrichtung** (60) zum Übertragen einer Kraft zu einer Branche (32) oder einem anderen bewegbaren Bauteil des Werkzeugs (30),
einem **Verriegelungsvorsprung** (68) an der Übertragungseinrichtung (60) zum Eingreifen in Schlitze (25, 86) oder Nute an einem in den Hohlraum (46) im ersten Bauteil (40) eingeführten distalen Ende (22) eines Schafts (20), zum Verriegeln einer Renkverbindung zwischen dem Werkzeug (30) und dem Schaft (20).

8. Werkzeug (30) nach dem vorangehenden Anspruch, bei dem die Übertragungseinrichtung (60) umfasst:
einen **Stab** (70) oder ein Rohr mit einem Schlitz (74) am distalen Ende (72) des Stabs (70) oder Rohrs;
einem **Endstück** (62) mit einem Lager (64) am distalen Ende des Endstücks (62) für eine gelenkige mechanische Kopplung mit einem bewegbaren Bauteil (32) des Werkzeugs (30),
wobei ein proximales Ende (66) des Endstücks (60) in den Schlitz (74) am distalen Ende (72) des Stabs (70) oder Rohrs eingesetzt und mit diesem gefügt ist,
wobei ein Bereich des proximalen Endes (66) des Endstücks (60), der seitlich aus dem Schlitz (74) am distalen Ende (72) des Stabs (70) oder Rohrs heraus ragt, den Verriegelungsvorsprung (68) bildet.

9. **Zerlegbares medizinisches Instrument** (10) mit:
einem **Schaft** (20);
einem **Werkzeug** (30) nach einem der vorangehenden Ansprüche.

10. Medizinisches Instrument (10) nach dem vorangehenden Anspruch, wobei der Schaft (20) umfasst:
ein **Schaftrohr** (24);
**L-förmige Schlitze** (25, 26) oder L-förmige Nuten am distalen Ende (22) des Schaftsrohrs (24);
ein **Stützrohr** (80) am distalen Ende (22) des Schafts (20) zum mechanischen Stützen und Verstärken des distalen Endes des Schaftrohrs (24),
wobei das **Stützrohr** (80) **innerhalb des Schaftrohrs** (24) angeordnet ist.

11. Medizinisches Instrument (10) nach dem vorangehenden Anspruch, bei dem das Stützrohr (80) **Schlitze** (86) oder Nuten **zur Aufnahme des Verriegelungsvorsprungs** (68) an der Übertragungseinrichtung (60) aufweist.

## Claims

1. Tool (30) for a dismantlable medical instrument (10), with:
a first component (40), which surrounds a cavity (46) for receiving a distal end (22) of a shank (20) ;
a second component (50) with several catches (56), which are distributed uniformly about the inner circumference of the cavity (46) and at equal angle distances from each other, which protrude radially into the cavity (46) from the surface of the cavity (46), which surface has the shape of the jacket of a circular cylinder, and are provided to form a bayonet connection and designed to engage from outside in slits (25, 26) or outwardly open grooves at the distal end of the shank (20) which protrudes into the cavity (46),
wherein the first component (40) has recesses (48) into which the second component (50) is fitted, wherein a radially outer end or a radially outer area (58) of each catch completely fills a respective recess (48) of the recesses (48).

2. Tool (30) according to the preceding claim, in which the first component (40) comprises a bearing (43) for the movable mounting of a branch (32) or of another movable component of the tool (30).

3. Tool (30) according to one of the preceding claims, in which the recesses (48) on the first component (40) are slits which are open at the proximal end, into which the catches (56) are inserted from the proximal direction, and in which the catches (56) are secured.

4. Tool (30) according to one of the preceding claims, in which the catches (56) are cohesively bonded to the edge of the recesses (48).

5. Tool (30) according to one of the preceding claims, in which
the second component (50) comprises the catches (56) and an annular area (54),
said annular area (54) being arranged proximally in relation to the catches (56).

6. Tool (30) according to the preceding claim, in which the annular area (54) of the second component (50) is joined to a proximal edge (44) of the first component (40).

7. Tool (10) according to one of the preceding claims, moreover with:
a transmission mechanism (60) for transmitting a force to a branch (32) or to another movable component of the tool (30),
a locking projection (68) on the transmission mechanism (60) for engaging in slits (25, 86) or grooves at a distal end (22) of a shank (20) inserted into the cavity (46) in the first component (40), in order to lock a bayonet connection between the tool (30) and the shank (20) .

8. Tool (30) according to the preceding claim, in which the transmission mechanism (60) comprises:
a rod (70) or a tube with a slit (74) at the distal end (72) of the rod (70) or tube;
an endpiece (62) with a bearing (64) at the distal end of the endpiece (62) for an articulated mechanical coupling to a movable component (32) of the tool (30),
wherein a proximal end (66) of the endpiece (60) is inserted into the slit (74) at the distal end (72) of the rod (70) or tube and is joined thereto,
wherein an area of the proximal end (66) of the endpiece (60), protruding laterally from the slit (74) at the distal end (72) of the rod (70) or tube, forms the locking projection (68).

9. Dismantlable medical instrument (10), with:
a shank (20) ;
a tool (30) according to one of the preceding claims.

10. Medical instrument (10) according to the preceding claim, wherein the shank (20) comprises:
a shank tube (24);
L-shaped slits (25, 26) or L-shaped grooves at the distal end (22) of the shank tube (24);
a support tube (80) at the distal end (22) of the shank (20), for mechanically supporting and reinforcing the distal end of the shank tube (24),
wherein the support tube (80) is arranged inside the shank tube (24).

11. Medical instrument (10) according to the preceding claim, in which the support tube (80) has slits (86) or grooves for receiving the locking projection (68) on the transmission mechanism (60) .

## Revendications

1. Outil (30) pour un instrument médical démontable (10), comportant :
un premier élément (40) qui entoure une cavité (46) destinée à recevoir une extrémité distale (22) d'une tige (20) ;
un deuxième élément (50) doté de plusieurs taquets (56) qui sont répartis sur la périphérie intérieure de la cavité (46) de manière uniforme ou suivant des écarts angulaires identiques, lesquels taquets font saillie dans la cavité (46) dans la direction radiale à partir de la surface en forme d'enveloppe cylindrique circulaire de la cavité (46) et sont prévus pour la formation d'une liaison à baïonnette et conçus pour venir en prise depuis l'extérieur dans des fentes (25, 26) ou des rainures ouvertes vers l'extérieur à l'extrémité distale de la tige (20) faisant saillie dans la cavité (46),
le premier élément (40) comprenant des évidements (48) dans lesquels le deuxième élément (50) est emboîté, une extrémité radialement extérieure ou une région radialement extérieure (58) de chaque taquet remplissant complètement un évidement (48) respectif parmi les évidements (48).

2. Outil (30) selon la revendication précédente, dans lequel le premier élément (40) comporte un palier (43) pour le montage mobile d'une branche (32) ou d'un autre élément mobile de l'outil (30).

3. Outil (30) selon l'une des revendications précédentes, dans lequel les évidements (48) sur le premier élément (40) sont des fentes ouvertes de manière proximale, dans lesquelles les taquets (56) sont insérés à partir de la direction proximale, et dans lesquelles les taquets (56) sont fixés.

4. Outil (30) selon l'une des revendications précédentes, dans lequel les taquets (56) sont reliés par liaison de matière au bord des évidements (48).

5. Outil (30) selon l'une des revendications précédentes, dans lequel
le deuxième élément (50) comporte les taquets (56) et une région annulaire (54),
la région annulaire (54) est disposée de manière proximale par rapport aux taquets (56).

6. Outil (30) selon la revendication précédente, dans lequel la région annulaire (54) du deuxième élément (50) est jointe à un bord proximal (44) du premier élément (40).

7. Outil (10) selon l'une des revendications précédentes, comportant en outre :
un dispositif de transmission (60) servant à la transmission d'une force à une branche (32) ou à un autre élément mobile de l'outil (30),
une saillie de verrouillage (68) sur le dispositif de transmission (60) destinée à venir en prise dans des fentes (25, 86) ou des rainures à une extrémité distale (22) d'une tige (20) insérée dans la cavité (46) dans le premier élément (40), pour le verrouillage d'une liaison à baïonnette entre l'outil (30) et la tige (20) .

8. Outil (30) selon la revendication précédente, dans lequel le dispositif de transmission (60) comporte :
une barre (70) ou un tube comportant une fente (74) à l'extrémité distale (72) de la barre (70) ou du tube ;
une pièce d'extrémité (62) comportant un palier (64) à l'extrémité distale de la pièce d'extrémité (62) pour un accouplement mécanique articulé à un élément mobile (32) de l'outil (30),
une extrémité proximale (66) de la pièce d'extrémité (60) étant insérée dans la fente (74) à l'extrémité distale (72) de la barre (70) ou du tube et étant jointe à celle-ci,
une région de l'extrémité proximale (66) de la pièce d'extrémité (60) formant la saillie de verrouillage (68), laquelle région fait saillie latéralement à partir de la fente (74) à l'extrémité distale (72) de la barre (70) ou du tube.

9. Instrument médical démontable (10) comportant :
une tige (20);
un outil (30) selon l'une des revendications précédentes.

10. Instrument médical (10) selon la revendication précédente, dans lequel la tige (20) comporte :
un tube de tige (24) ;
des fentes (25, 26) en forme de L ou des rainures en forme de L à l'extrémité distale (22) du tube de tige (24) ;
un tube de support (80) à l'extrémité distale (22) de la tige (20) pour le support mécanique et le renforcement de l'extrémité distale du tube de tige (24),
le tube de support (80) étant disposé à l'intérieur du tube de tige (24).

11. Instrument médical (10) selon la revendication précédente, dans lequel le tube de support (80) comprend des fentes (86) ou des rainures destinées à recevoir la saillie de verrouillage (68) sur le dispositif de transmission (60).
